# EUROPÄISCHE PATENTANMELDUNG

(11) **EP 1 449 847 A1**
(43) Veröffentlichungstag der Anmeldung: **25.08.2004**
(21) Anmeldenummer: 04003127.0
(22) Anmeldetag: 12.02.2004
(51) Int. Cl.: C07J 63/00, A61K 31/56, A61P 17/10

(54) **Glyzyrrhetinsäureester als Anti-Aknemittel**

(30) Priorität: 21.02.2003 DE 10307388
(71) Anmelder: Cognis Deutschland GmbH & Co. KG, 40589 Düsseldorf (DE)
(72) Erfinder: Mehling, Annette, Dr., 42349 Wuppertal (DE); Fabry, Bernd, Dr., 41352 Korschenbroich (DE)

(57) **Zusammenfassung**

Vorgeschlagen werden Ester der Glyzyrrhetinsäure gemäß Formel (I), 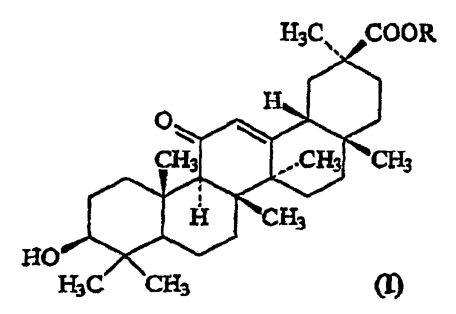 in der R für den Rest eines aromatischen Alkohols steht, als Anti-Aknemittel.

## Beschreibung

### Gebiet der Erfindung

Die Erfindung befindet sich auf dem Gebiet der pharmazeutischen Wirkstoffe und betrifft neue Glyzyrrhetinsäureester, Anti-Aknemittel auf Basis dieser Ester sowie deren Verwendung zur Herstellung von kosmetischen, pharmazeutischen und dermatologischen Zubereitungen.

### Stand der Technik

Unter dem Begriff Akne versteht der Fachmann eine Hauterkrankung, die sich durch entzündliche und nicht entzündliche Knötchen auszeichnen und die zur Bildung von Pusteln, Abszessen und schließlich Narben führen kann. Obschon die Ursachen unterschiedlich sind, lässt sich Akne letztlich auf verstopfte Haarfollikel (Komedone) zurückführen. Neben einer hormonell bedingten Verstopfung der Haarfollikel-Mündungen durch Körperfette, besteht eine wesentliche Ursache für die Entstehung von Akne in der Entwicklung gewebeschädigender freier Fettsäuren und Enzyme durch Bakterien, wie beispielsweise Propionibacterium acnes.

Aus dem Stand der Technik sind eine ganze Reihe von Wirkstoffen bekannt, die zur Bekämpfung von Akne mit mehr oder minder großem Erfolg eingesetzt werden können. So wird beispielsweise in der Spanischen Patentschrift **ES 9702410 B1** (Vinyals) die Verwendung des Zinksalzes der Glyzyrrhetinsäure gegen Akne vorgeschlagen. Aus den beiden Europäischen Patentschriften **EP 0336880 B1** und **EP 0443413 B1** (Schering) ist die Verwendung von Azelainsäure, deren Salzen und Estern für den gleichen Zweck bekannt. Von Tamarkin werden in der internationalen Patentanmeldung **WO 98/020834 A2** zur Bekämpfung von dermatologischen Erkrankungen Ester von Dicarbonsäuren mit "keratolytisch aktiven" Alkoholen vorgeschlagen. Als weitere Wirkstoffe, die im wesentlichen gegen eine überhöhte Sebumproduktion wirksam sind, seien beispielsweise Benzoylperoxid, Hexaclorophen, Dodecylbenzolsulfonsäure, 2,2',2'-Nitrilotriethanol-Salze, Dexpanthenol oder Resorcin genannt. Keiner dieser Stoffe zeigt jedoch für sich alleine genommen eine befriedigende Wirkung gegenüber den Keimen, die maßgeblich am Entstehen von Akne, speziell von Acne vulgaris, beteiligt sind.

Die Aufgabe der Erfindung hat daher darin bestanden, neue Wirkstoffe zur Verfügung zu stellen, die einerseits gegenüber den am Entstehen von Akne beteiligten Bakterien eine verbesserte Wirkung aufweisen und zudem den Heilungsprozess vorzugsweise durch antiinflammatorische Eigenschaften unterstützen.

### Beschreibung der Erfindung

Gegenstand der Erfindung sind Ester der Glyzyrrhetinsäure gemäß Formel **(I)**, 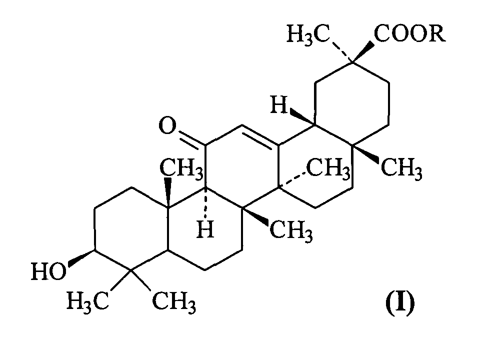 in der R für den Rest eines aromatischen Alkohols steht, wobei sich dieser Alkohol vorzugsweise ableitet von Benzylalkohol, Salicylalkohol, Sterol, Tocopherol, Retinol sowie deren Gemischen.

Überraschenderweise wurde gefunden, dass die aromatischen Ester der Glyzyrrhetinsäure, insbesondere die Glyzyrrhetinsäuresalicylate und -retinate eine besonders hohe bakterzide Wirkung aufweisen und dabei gleichzeitig anti-inflammatorisch wirken, d.h. die Wundheilung beschleunigen. Ursache dafür könnte der gegenüber der Säure oder deren Salzen erleichterte Transport der Wirkstoffe durch die Lipidbarriere des stratum corneums sein, wobei dann die Säure durch enzymatische Spaltung in den oberen Hautschichten, d.h. direkt vor dem Ort der Entzündung freigesetzt wird. Die Erfindung schließt die Erkenntnis ein, dass auch die Mischungen von freier Glyzyrrhetinsäure und aromatischem Alkohol, insbesondere wiederum dann, wenn es sich dabei um Salicylalkohol oder Retinol handelt, eine synergistische Anti-Aknewirkung zeigen.

### Anti-Aknemittel

Ein weiterer Gegenstand der vorliegenden Erfindung betrifft, Anti-Aknemittel, die einen wirksamen Gehalt der neuen Glyzyrrhetinsäureester enthalten. Glyzyrrhetinsäure findet sich in Extrakten der *Glycyrrhiza glabra;* Derivate mit einer 1,2-β-Zuckerbindung sind beispielsweise für den Lakritzgeschmack verantwortlich. Die Herstellung der Ester kann in an sich bekannter Weise erfolgen. In diesem Zusammenhang sei beispielsweise auf die Britische Patentanmeldung **GB-A 2 285805** (Marigen) verwiesen, in der detailliert die Herstellung von Steroidestern sowie deren Verwendung gegen Hautkrebs beschrieben wird.

In einer bevorzugten Ausführungsform der Erfindung enthalten die Mittel weitere Wirkstoffe, die ausgewählt sind aus der Gruppe, die gebildet wird von
- Glyzyrrhetinsäure und deren Salzen oder Extrakten der Pflanze *Glyzyrrhiza glabra,*
- Azelainsäure, deren Salzen und Estern,
- Salicylsäure, deren Salzen und Estern,
- Benzoylperoxid,
- Vitamin B6
- Vitamin E phosphat (z.B. Vitol® IEP) sowie
- Pflanzenextrakten.

Beispiele für Derivate der Glyzyrrhetinsäure, Salicylsäure bzw. Azelainsäure sind deren Alkali-, Erdalkali-, Ammonium-, Alkylammonium-, Alkanolammonium-, Glucammonium- und Zinksalze sowie (soweit beansprucht) deren Ester mit linearen oder verzweigten aliphatischen Alkoholen mit 1 bis 18 Kohlenstoffatomen sowie die Voll- oder Partialester der Säuren mit Polyolen mit 2 bis 15 Kohlenstoffatomen und mindestens 2 Hydroxylgruppen in Frage.

Typische Beispiele sind die Natrium-, Kalium-, Ammonium-, Triethanolammonium-, Glucammonium und Zinksalze der Glyzyrrhetinsäure, Azelainsäure und Salicylsäure, die Ester der Salicylsäure oder Azelainsäure mit Methanol, Ethanol, den isomeren Propanolen und Butanolen sowie Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol und deren Gemischen. Weitere Beispiele sind die Voll- oder Partialester der Salicylsäure oder Azelainsäure mit Glycerin; Alkylenglycolen, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton; technischen Oligoglyceringemischen mit einem Eigenkondensationsgrad von 1,5 bis 10 wie etwa technische Diglyceringemische mit einem Diglyceringehalt von 40 bis 50 Gew.-%; Methyolverbindungen, wie insbesondere Trimethylolethan, Trimethylolpropan, Trimethylolbutan, Pentaerythrit und Dipentaerythrit; Niedrigalkylglucosiden, insbesondere solche mit 1 bis 8 Kohlenstoffen im Alkylrest, wie beispielsweise Methyl- und Butylglucosid; Zuckeralkoholen mit 5 bis 12 Kohlenstoffatomen, wie beispielsweise Sorbit oder Mannit; Zuckern mit 5 bis 12 Kohlenstoffatomen, wie beispielsweise Glucose oder Saccharose; Aminozuckern, wie beispielsweise Glucamin; oder Dialkoholaminen, wie Diethanolamin oder 2-Amino-1,3-propandiol.

Typische Beispiele für geeignete Pflanzenextrakte sind die Wirkstoffe folgender Pflanzen *: Aesculus hippocastanum* (Rosskastanie), *Argania spinosa, Babtista tinctoria* (wilder Indigo), *Cantella asiatica, Camelilla sinensis* (Grüner Tee), *Chamonella recutita* (Kamille), *Ginkgo biloba* (Ginkgo), *Oleo europea* (Olive), *Litschi chinensis* (Litchi), *Melissa officinalis* (Zitronenmelisse), *Panax ginseng* (Ginseng), *Passiflora incarnata* (Passionsblume), *Prunus dulcis* (Süßmandel), *Pterocarpus marsupium, Ruscus aculeatus, Trifolium pratense* (Red Clover), *Uva ursi* (Bärtraube), *Vaccinium myrtillus* (Blaubeere), *Vigna acontifolia,* und *Vitis vinifera* (wilder Wein).

Die Endzubereitungen können die Glyzyrrhetinsäureester und die weiteren Wirkstoffe im Gewichtsverhältnis 90 : 10 bis 10 : 90 enthalten, wobei beträchtliche Synergien im Bereich von 75 : 25 bis 25 : 75 und insbesondere 60 : 40 bis 40 : 60 festgestellt werden.

### Mikrokapseln und Pro-Liposomen

In einer besonderen Ausfühungsform der vorliegenden Erfindung können die Wirkstoffe in Form von Mikrokapseln oder Pro-Liposmen vorliegen. Unter dem Begriff "Mikrokapsel" werden vom Fachmann sphärische Aggregate mit einem Durchmesser im Bereich von etwa 0,0001 bis etwa 5 mm verstanden, die mindestens einen festen oder flüssigen Kern enthalten, der von mindestens einer kontinuierlichen Hülle umschlossen ist. Genauer gesagt handelt es sich um mit filmbildenden Polymeren umhüllte feindisperse flüssige oder feste Phasen, bei deren Herstellung sich die Polymere nach Emulgierung und Koazervation oder Grenzflächenpolymerisation auf dem einzuhüllenden Material niederschlagen. Nach einem anderen Verfahren werden geschmolzene Wachse in einer Matrix aufgenommen ("microsponge"), die als Mikropartikel zusätzlich mit filmbildenden Polymeren umhüllt sein können. Die mikroskopisch kleinen Kapseln, auch Nanokapseln genannt, lassen sich wie Pulver trocknen. Neben einkernigen Mikrokapseln sind auch mehrkernige Aggregate, auch Mikrosphären genannt, bekannt, die zwei oder mehr Kerne im kontinuierlichen Hüllmaterial verteilt enthalten. Ein- oder mehrkernige Mikrokapseln können zudem von einer zusätzlichen zweiten, dritten etc. Hülle umschlossen sein. Die Hülle kann aus natürlichen, halbsynthetischen oder synthetischen Materialien bestehen. Natürlich Hüllmaterialien sind beispielsweise Gummi Arabicum, Agar-Agar, Agarose, Maltodextrine, Alginsäure bzw. ihre Salze, z.B. Natrium- oder Calciumalginat, Fette und Fettsäuren, Cetylalkohol, Collagen, Chitosan, Lecithine, Gelatine, Albumin, Schellack, Polysaccharide, wie Stärke oder Dextran, Polypeptide, Proteinhydrolysate, Sucrose und Wachse. Halbsynthetische Hüllmaterialien sind unter anderem chemisch modifizierte Cellulosen, insbesondere Celluloseester und -ether, z.B. Celluloseacetat, Ethylcellulose, Hydroxypropylcellulose, Hydroxypropylmethylcellulose und Carboxymethylcellulose, sowie Stärkederivate, insbesondere Stärkeether und -ester. Synthetische Hüllmaterialien sind beispielsweise Polymere wie Polyacrylate, Polyamide, Polyvinylalkohol oder Polyvinylpyrrolidon.

Beispiele für Mikrokapseln des Stands der Technik sind folgende Handelsprodukte (in Klammern angegeben ist jeweils das Hüllmaterial) : *Hallcrest Microcapsules* (Gelatine, Gummi Arabicum), *Coletica Thalaspheres* (maritimes Collagen), *Lipotec Millicapseln* (Alginsäure, Agar-Agar), *Induchem Unispheres* (Lactose, mikrokristalline Cellulose, Hydroxypropylmethylcellulose); *Unicerin C30* (Lactose, mikrokristalline Cellulose, Hydroxypropylmethylcellulose), *Kobo Glycospheres* (modifizierte Stärke, Fettsäureester, Phospholipide), *Softspheres* (modifiziertes Agar-Agar) und *Kuhs Probiol Nanospheres* (Phospholipide) sowie *Primaspheres* und *Primasponges* (Chitosan, Alginate) und *Primasys* (Phospholipide).

Chitosanmikrokapseln und Verfahren zu ihrer Herstellung sind Gegenstand früherer Patenanmeldungen der Patentanmelderin **[WO 01/01926, WO 01/01927, WO 01/01928, WO 01/01929].**

Zur Herstellung derartiger Mikrokapseln mit mittleren Durchmessern im Bereich von 0,0001 bis 5, vorzugsweise 0,001 bis 0,5 und insbesondere 0,005 bis 0,1 mm, bestehend aus einer Hüllmembran und einer die Wirkstoffe enthaltenden Matrix, geht man vorzugsweise so vor, dass man
(a1) aus Gelbildnern, Chitosanen und den Wirkstoffen eine Matrix zubereitet,
(a2) gegebenenfalls die Matrix in einer Ölphase dispergiert,
(a3) die gegebenenfalls dispergierte Matrix mit wässrigen Lösungen anionischer Polymere behandelt und gegebenenfalls dabei die Ölphase entfernt.
oder
(b1) aus Gelbildnern, anionischen Polymeren und den Wirkstoffen eine Matrix zubereitet,
(b2) gegebenenfalls die Matrix in einer Ölphase dispergiert,
(b3) die gegebenenfalls dispergierte Matrix mit wässrigen Chitosanlösungen behandelt und gegebenenfalls dabei die Ölphase entfernt.
oder
(c1) wässrige Zubereitungen der Wirkstoffe mit Ölkörpern in Gegenwart von Emulgatoren zu O/W-Emulsionen verarbeitet,
(c2) die so erhaltenen Emulsionen mit wässrigen Lösungen anionischer Polymere behandelt,
(c3) die so erhaltene Matrix mit wässrigen Chitosanlösungen in Kontakt bringt und
(c4) die so erhaltenen Verkapselungsprodukte von der wässrigen Phase abtrennt.

### Gelbildner

Im Sinne der Erfindung werden als Gelbildner vorzugsweise solche Stoffe in Betracht gezogen, welche die Eigenschaft zeigen in wässriger Lösung bei Temperaturen oberhalb von 40 °C Gele zu bilden. Typische Beispiele hierfür sind Heteropolysaccharide und Proteine. Als thermogelierende Heteropolysaccharide kommen vorzugsweise Agarosen in Frage, welche in Form des aus Rotalgen zu gewinnenden Agar-Agar auch zusammen mit bis zu 30 Gew.-% nicht-gelbildenden Agaropektinen vorliegen können. Hauptbestandteil der Agarosen sind lineare Polysaccharide aus D-Galaktose und 3,6-Anhydro-L-galaktose, die alternierend β-1,3- und β-1,4-glykosidisch verknüpft sind. Die Heteropolysaccharide besitzen vorzugsweise ein Molekulargewicht im Bereich von 110.000 bis 160.000 und sind sowohl farb- als auch geschmacklos: Als Alternativen kommen Pektine, Xanthane (auch Xanthan Gum) sowie deren Mischungen in Frage. Es sind weiterhin solche Typen bevorzugt, die noch in 1-Gew.-%iger wässriger Lösung Gele bilden, die nicht unterhalb von 80 °C schmelzen und sich bereits oberhalb von 40 °C wieder verfestigen. Aus der Gruppe der thermogelierenden Proteine seien exemplarisch die verschiedenen Gelatine-Typen genannt.

### Chitosane

Chitosane stellen Biopolymere dar und werden zur Gruppe der Hydrokolloide gezählt. Chemisch betrachtet handelt es sich um partiell deacetylierte Chitine unterschiedlichen Molekulargewichtes, die den folgenden - idealisierten - Monomerbaustein enthalten:

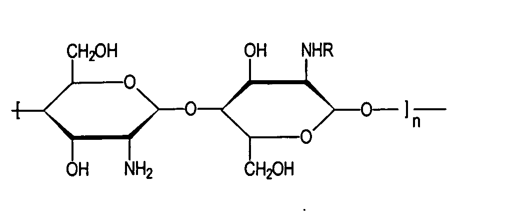

Im Gegensatz zu den meisten Hydrokolloiden, die im Bereich biologischer pH-Werte negativ geladen sind, stellen Chitosane unter diesen Bedingungen kationische Biopolymere dar. Die positiv geladenen Chitosane können mit entgegengesetzt geladenen Oberflächen in Wechselwirkung treten und werden daher in kosmetischen Haar- und Körperpflegemitteln sowie pharmazeutischen Zubereitungen eingesetzt. Zur Herstellung der Chitosane geht man von Chitin, vorzugsweise den Schalenresten von Krustentieren aus, die als billige Rohstoffe in großen Mengen zur Verfügung stehen. Das Chitin wird dabei in einem Verfahren, das erstmals von Hackmann et al. beschrieben worden ist, üblicherweise zunächst durch Zusatz von Basen deproteiniert, durch Zugabe von Mineralsäuren demineralisiert und schließlich durch Zugabe von starken Basen deacetyliert, wobei die Molekulargewichte über ein breites Spektrum verteilt sein können. Vorzugsweise werden solche Typen eingesetzt, wie die ein durchschnittliches Molekulargewicht von 10.000 bis 500.000 bzw. 800.000 bis 1.200.000 Dalton aufweisen und/oder eine Viskosität nach Brookfield (1 Gew.-%ig in Glykolsäure) unterhalb von 5000 mPas, einen Deacetylierungsgrad im Bereich von 80 bis 88 % und einem Aschegehalt von weniger als 0,3 Gew.-% besitzen. Aus Gründen der besseren Wasserlöslichkeit werden die Chitosane in der Regel in Form ihrer Salze, vorzugsweise als Glycolate eingesetzt.

### Ölphase

Die Matrix kann vor der Bildung der Membran optional in einer Ölphase dispergiert werden. Als Öle kommen für diesen Zweck beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen C₆-C₂₂-Fettalkoholen, Ester von verzweigten C₆-C₁₃-Carbonsäuren mit linearen C₆-C₂₂-Fettalkoholen, wie z.B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, I-sostearylisostearat, Isostearyloleat, Isostearylbehenat, Isostearyloleat, Oleylmyristat, O-leylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Daneben eignen sich Ester von linearen C₆-C₂₂-Fettsäuren mit verzweigten Alkoholen, insbesondere 2-Ethylhexanol, Ester von Hydroxycarbonsäuren mit linearen oder verzweigten C₆-C₂₂-Fettalkoholen, insbesondere Dioctyl Malate, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalkoholcarbonate, Guerbetcarbonate, Ester der Benzoesäure mit linearen und/oder verzweigten C₆-C₂₂-Alkoholen (z.B. Finsolv® TN), lineare oder verzweigte, symmetrische oder unsymmetrische Dialkylether mit 6 bis 22 Kohlenstoffatomen pro Alkylgruppe, Ringöffnungsprodukte von epoxidierten Fettsäureestern mit Polyolen, Siliconöle und/oder aliphatische bzw. naphthenische Kohlenwasserstoffe, wie z.B. wie Squalan, Squalen oder Dialkylcyclohexane in Betracht.

### Anionpolymere

Die anionische Polymere haben die Aufgabe, mit den Chitosanen Membranen zu bilden. Für diesen Zweck eignen sich vorzugsweise Salze der Alginsäure. Bei der Alginsäure handelt es sich um ein Gemisch carboxylgruppenhaltiger Polysaccharide mit folgendem idealisierten Monomerbaustein:

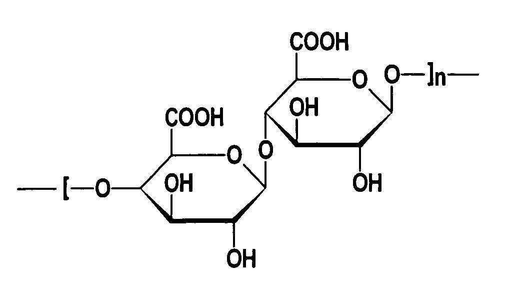

Das durchschnittliche Molekulargewicht der Alginsäuren bzw. der Alginate liegt im Bereich von 150.000 bis 250.000. Dabei sind als Salze der Alginsäure sowohl deren vollständige als auch deren partiellen Neutralisationsprodukte zu verstehen, insbesondere die Alkalisalze und hierunter vorzugsweise das Natriumalginat ("Algin") sowie die Ammonium- und Erdalkalisalze. besonders bevorzugt sind Mischalginate, wie z.B. Natrium/Magnesium- oder Natrium/Calciumalginate. In einer alternativen Ausführungsform der Erfindung kommen für diesen Zweck jedoch auch anionische Chitosanderivate, wie z.B. Carboxylierungs- und vor allem Succinylierungsprodukte in Frage. Alternativ kommen auch Poly(meth)acrylate mit durchschnittlichen Molekulargewichten im Bereich von 5.000 bis 50.000 Dalton sowie die verschiedenen Carboxymethylcellulosen in Frage. Anstelle der anionischen Polymeren können für die Ausbildung der Hüllmembran auch anionische Tenside oder niedermolekulare anorganische Salze, wie beispielsweise Pyrophosphate eingesetzt werden.

### Emulgatoren

Als Emulgatoren kommen beispielsweise nichtionogene Tenside aus mindestens einer der folgenden Gruppen in Frage:
- Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe sowie Alkylamine mit 8 bis 22 Kohlenstoffatomen im Alkylrest;
- Alkyl- und/oder Alkenyloligoglykoside mit 8 bis 22 Kohlenstoffatomen im Alk(en)ylrest und deren ethoxylierte Analoga;
- Anlagerungsprodukte von 1 bis 15 Mol Ethylenoxid an Ricinusöl und/oder gehärtetes Ricinusöl;
- Anlagerungsprodukte von 15 bis 60 Mol Ethylenoxid an Ricinusöl und/oder gehärtetes Ricinusöl;
- Partialester von Glycerin und/oder Sorbitan mit ungesättigten, linearen oder gesättigten, verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
- Partialester von Polyglycerin (durchschnittlicher Eigenkondensationsgrad 2 bis 8), Polyethylenglycol (Molekulargewicht 400 bis 5000), Trimethylolpropan, Pentaerythrit, Zuckeralkoholen (z.B. Sorbit), Alkylglucosiden (z.B. Methylglucosid, Butylglucosid, Laurylglucosid) sowie Polyglucosiden (z.B. Cellulose) mit gesättigten und/oder ungesättigten, linearen oder verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
- Mischester aus Pentaerythrit, Fettsäuren, Citronensäure und Fettalkohol und/oder Mischester von Fettsäuren mit 6 bis 22 Kohlenstoffatomen, Methylglucose und Polyolen, vorzugsweise Glycerin oder Polyglycerin.
- Mono-, Di- und Trialkylphosphate sowie Mono-, Di- und/oder Tri-PEGalkylphosphate und deren Salze;
- Wollwachsalkohole;
- Polysiloxan-Polyalkyl-Polyether-Copolymere bzw. entsprechende Derivate;
- Block-Copolymere z.B. Polyethylenglycol-30 Dipolyhydroxystearate;
- Polymeremulgatoren, z.B. Pemulen-Typen (TR-1,TR-2) von Goodrich;
- Polyalkylenglycole sowie
- Glycerincarbonat.

### • Ethylenoxidanlagerungsprodukte

Die Anlagerungsprodukte von Ethylenoxid und/oder von Propylenoxid an Fettalkohole, Fettsäuren, Alkylphenole oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/ oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind als Rückfettungsmittel für kosmetische Zubereitungen bekannt.

### • Alkyl- und/oder Alkenyloligoglykoside

Alkyl- und/oder Alkenyloligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 18 Kohlenstoffatomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

### • Partialglyceride

Typische Beispiele für geeignete Partialglyceride sind Hydroxystearinsäuremonoglycerid, Hydroxystearinsäurediglycerid, Isostearinsäuremonoglycerid, Isostearinsäurediglycerid, Ölsäuremonoglycerid, Ölsäurediglycerid, Ricinolsäuremoglycerid, Ricinolsäurediglycerid, Linolsäuremonoglycerid, Linolsäurediglycerid, Linolensäuremonoglycerid, Linolensäurediglycerid, Erucasäuremonoglycerid, Erucasäurediglycerid, Weinsäuremonoglycerid, Weinsäurediglycerid, Citronensäuremonoglycerid, Citronendiglycerid, Äpfelsäuremonoglycerid, Äpfelsäurediglycerid sowie deren technische Gemische, die untergeordnet aus dem Herstellungsprozeß noch geringe Mengen an Triglycerid enthalten können. Ebenfalls geeignet sind Anlagerungsprodukte von 1 bis 30, vorzugsweise 5 bis 10 Mol Ethylenoxid an die genannten Partialglyceride.

### • Sorbitanester

Als Sorbitanester kommen Sorbitanmonoisostearat, Sorbitansesquiisostearat, Sorbitan-diisostearat, Sorbitantriisostearat, Sorbitanmonooleat, Sorbitansesquioleat, Sorbitan-dioleat, Sorbitantrioleat, Sorbitanmonoerucat, Sorbitansesquierucat, Sorbitandierucat, Sorbitantrierucat, Sorbitanmonoricinoleat, Sorbitansesquiricinoleat, Sorbitandiricinoleat, Sorbitantriricinoleat, Sorbitanmonohydroxystearat, Sorbitansesquihydroxystearat, Sorbitandihydroxystearat, Sorbitantrihydroxystearat, Sorbitanmonotartrat, Sorbitansesquitartrat, Sorbitanditartrat, Sorbitantritartrat, Sorbitanmonocitrat, Sorbitansesquicitrat, Sorbitandicitrat, Sorbitantricitrat, Sorbitanmonomaleat, Sorbitansesquimaleat, Sorbitandimaleat, Sorbitantrimaleat sowie deren technische Gemische. Ebenfalls geeignet sind Anlagerungsprodukte von 1 bis 30, vorzugsweise 5 bis 10 Mol Ethylenoxid an die genannten Sorbitanester.

### • Polyglycerinester

Typische Beispiele für geeignete Polyglycerinester sind Polyglyceryl-2 Dipolyhydroxystearate (Dehymuls® PGPH), Polyglycerin-3-Diisostearate (Lameform® TGI), Polyglyceryl-4 Isostearate (Isolan® GI 34), Polyglyceryl-3 Oleate, Diisostearoyl Polyglyceryl-3 Diisostearate (Isolan® PDI), Polyglyceryl-3 Methylglucose Distearate (Tego Care® 450), Polyglyceryl-3 Beeswax (Cera Bellina®), Polyglyceryl-4 Caprate (Polyglycerol Caprate T2010/90), Polyglyceryl-3 Cetyl Ether (Chimexane® NL), Polyglyceryl-3 Distearate (Cremophor® GS 32) und Polyglyceryl Polyricinoleate (Admul® WOL 1403) Polyglyceryl Dimerate Isostearate sowie deren Gemische. Beispiele für weitere geeignete Polyolester sind die gegebenenfalls mit 1 bis 30 Mol Ethylenoxid umgesetzten Mono-, Di- und Triester von Trimethylolpropan oder Pentaerythrit mit Laurinsäure, Kokosfettsäure, Talgfettsäure, Palmitinsäure, Stearinsäure, Ölsäure, Behensäure und dergleichen.

### • Anionische Emulgatoren

Typische anionische Emulgatoren sind aliphatische Fettsäuren mit 12 bis 22 Kohlenstoffatomen, wie beispielsweise Palmitinsäure, Stearinsäure oder Behensäure, sowie Dicarbonsäuren mit 12 bis 22 Kohlenstoffatomen, wie beispielsweise Azelainsäure oder Sebacinsäure.

### • Amphotere und kationische Emulgatoren

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethyl-ammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl Betaine* bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder Acylgruppe im Molekül mindestens eine freie Aminogruppe und mindestens eine -COOH- oder-SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe.. Besonders bevorzugte ampholytische Tenside sind das N-Kokosalkylaminopropionat, das Kokosacylaminoethylaminopropionat und das C_{12/18}-Acylsarcosin. Schließlich kommen auch Kationtenside als Emulgatoren in Betracht, wobei solche vom Typ der Esterquats, vorzugsweise methylquaternierte Difettsäuretriethanolaminester-Salze, besonders bevorzugt sind.

### Herstellverfahren Mikrokapseln

Zur Herstellung der Mikrokapseln stellt man üblicherweise eine 1 bis 10, vorzugsweise 2 bis 5 Gew.-%ige wässrige Lösung des Gelbildners, vorzugsweise des Agar-Agars her und erhitzt diese unter Rückfluss. In der Siedehitze, vorzugsweise bei 80 bis 100°C, wird eine zweite wässrige Lösung zugegeben, welche das Chitosan in Mengen von 0,1 bis 2, vorzugsweise 0,25 bis 0,5 Gew.-% und den Wirkstoffen in Mengen von 0,1 bis 25 und insbesondere 0,25 bis 10 Gew.-% enthält; diese Mischung wird als Matrix bezeichnet. Die Beladung der Mikrokapseln mit Wirkstoffen kann daher ebenfalls 0,1 bis 25 Gew.-% bezogen auf das Kapselgewicht betragen. Falls gewünscht, können zu diesem Zeitpunkt zur Viskositätseinstellung auch wasserunlösliche Bestandteile, beispielsweise anorganische Pigmente zugegeben werden, wobei man diese in der Regel in Form von wässrigen oder wässrig/alkoholischen Dispersionen zusetzt. Zur Emulgierung bzw. Dispergierung der Wirkstoffe kann es ferner von Nutzen sein, der Matrix Emulgatoren und/oder Lösungsvermittler hinzuzugeben. Nach der Herstellung der Matrix aus Gelbildner, Chitosan und Wirkstoffmischung kann die Matrix optional in einer Ölphase unter starker Scherung sehr fein dispergiert werden, um bei der nachfolgenden Verkapselung möglichst kleine Teilchen herzustellen. Dabei hat es sich als besonders vorteilhaft erwiesen, die Matrix auf Temperaturen im Bereich von 40 bis 60 °C zu erwärmen, während man die Ölphase auf 10 bis 20 °C kühlt. Im letzten, nun wieder obligatorischen Schritt erfolgt dann die eigentliche Verkapselung, d.h. die Ausbildung der Hüllmembran durch Inkontaktbringen des Chitosans in der Matrix mit den anionischen Polymeren. Hierzu empfiehlt es sich, die gegebenenfalls in der Ölphase dispergierte Matrix bei einer Temperatur im Bereich von 40 bis 100, vorzugsweise 50 bis 60 °C mit einer wässrigen, etwa 1 bis 50 und vorzugsweise 10 bis 15 Gew.-%ige wässrigen Lösung des Anionpolymers zu behandeln und dabei - falls erforderlich - gleichzeitig oder nachträglich die Ölphase zu entfernen. Die dabei resultierenden wässrigen Zubereitungen weisen in der Regel einen Mikrokapselgehalt im Bereich von 1 bis 10 Gew.-% auf. In manchen Fällen kann es dabei von Vorteil sein, wenn die Lösung der Polymeren weitere Inhaltsstoffe, beispielsweise Emulgatoren oder Konservierungsmittel enthält. Nach Filtration werden Mikrokapseln erhalten, welche im Mittel einen Durchmesser im Bereich von vorzugsweise etwa 1 mm aufweisen. Es empfiehlt sich, die Kapseln zu sieben, um eine möglichst gleichmäßige Größenverteilung sicherzustellen. Die so erhaltenen Mikrokapseln können im herstellungsbedingten Rahmen eine beliebige Form aufweisen, sie sind jedoch bevorzugt näherungsweise kugelförmig. Alternativ kann man die Anionpolymere auch zur Herstellung der Matrix einsetzen und die Verkapselung mit den Chitosanen durchführen.

In einem alternativen Verfahren wird zur Herstellung der erfindungsgemäßen Mikrokapseln wird zunächst eine O/W-Emulsion zubereitet, welche neben dem Ölkörper, Wasser und den Wirkstoffen eine wirksame Menge Emulgator enthält. Zur Herstellung der Matrix wird diese Zubereitung unter starkem Rühren mit einer entsprechenden Menge einer wässrigen Anionpolymerlösung versetzt. Die Membranbildung erfolgt durch Zugabe der Chitosanlösung. Der gesamte Vorgang findet vorzugsweise im schwach sauren Bereich bei pH = 3 bis 4 statt. Falls erforderlich erfolgt die pH-Einstellung durch Zugabe von Mineralsäure. Nach der Membranbildung wird der pH-Wert auf 5 bis 6 angehoben, beispielsweise durch Zugabe von Triethanolamin oder einer anderen Base. Hierbei kommt es zu einem Anstieg der Viskosität, die durch Zugabe von weiteren Verdickungsmitteln, wie z.B. Polysacchariden, insbesondere Xanthan-Gum, Guar-Guar, Agar-Agar, Alginaten und Tylosen, Carboxymethylcellulose und Hydroxyethylcellulose, höhermolekularen Polyethylenglycolmono- und -diesten von Fettsäuren, Polyacrylaten, Polyacrylamiden und dergleichen noch unterstützt werden kann. Abschließend werden die Mikrokapseln von der wässrigen Phase beispielsweise durch Dekantieren, Filtrieren oder Zentrifugieren abgetrennt.

### Pro-Liposomen

Anstelle der beschriebenen Mikrokapseln kommen als Träger für die Wirkstoffgemische auch Pro-Liposomen in Frage. Zur Begriffsklärung sei darauf hingewiesen, dass die Pro-Liposomen kein Wasser enthalten und dieses erst dann unter Bildung von echten Liposomen aufnehmen, wenn sie in eine wässrige Umgebung eingebracht werden. Unter der Bezeichnung Lecithine versteht der Fachmann diejenigen Glycero-Phospholipide, die sich aus Fettsäuren, Glycerin, Phosphorsäure und Cholin durch Veresterung bilden. Lecithine werden in der Fachwelt daher auch häufig als Phosphatidylcholine (PC) bezeichnet. Als Beispiele für natürliche Lecithine, die zur Verkapselung in Frage kommen, seien die Kephaline genannt, die auch als Phosphatidsäuren bezeichnet werden und Derivate der 1,2-Diacyl-sn-glycerin-3-phosphorsäuren darstellen. Dem gegenüber versteht man unter Phospholipiden gewöhnlich Mono- und vorzugsweise Diester der Phosphorsäure mit Glycerin (Glycerinphosphate), die allgemein zu den Fetten gerechnet werden. Daneben kommen auch zur liposomalen Verkapselung Sphingosine bzw. Sphingolipide in Frage.

Pro-Liposomen können im Sinne der Erfindung erhalten werden, indem man die Wirkstoffe in pharmazeutisch bzw. kosmetisch akzeptablen Lösemitteln mit Lecithinen und/oder Phospholipiden behandelt. Hierzu werden die Wirkstoffe üblicherweise entweder in einem Lösungsmittel vorgelegt und mit den Lecithinen bzw. Phospholipiden bei Temperaturen im Bereich von 30 bis 70 °C in Kontakt gebracht oder aber die wasserfreien Gemische werden in eine Lösung der Lecithine bzw. Phospholipide eingerührt. Die Wirkstoffe und die Lecithine und/oder Phospholipide können dabei im Gewichtsverhältnis 1 : 20 bis 5:1, vorzugsweise 1 : 2 bis 4 : 1 eingesetzt werden. Als Lösemittel eignen sich vorzugsweise niedere Alkohole mit 1 bis 4 Kohlenstoffatome, wie z.B. Ethanol oder Polyole, welche in der Regel 2 bis 15 Kohlenstoffatome und mindestens zwei Hydroxylgruppen besitzen; hier ist Propylenglycol bevorzugt.

### Gewerbliche Anwendbarkeit

Ein weiterer Gegenstand der vorliegenden Erfindung betrifft die Verwendung der neuen Glyzyrrhetinsäureester zur Herstellung von kosmetischen, pharmazeutischen und/oder dermatologischen Zubereitungen, in denen sie in Mengen von 0,01 bis 20, vorzugsweise 0,1 bis 5 und insbesondere 0,5 bis 1 Gew.-% - bezogen auf die Endzubereitungen - enthalten sein können.

### Beispiele

### Herstellbeispiele

**Beispiel 1.** In einem 500-ml-Dreihalskolben mit Rührer und Rückflusskühler wurden in der Siedehitze 3 g Agar-Agar in 200 ml Wasser gelöst. Anschließend wurde die Mischung innerhalb von etwa 30 min unter starkem Rühren zunächst mit einer homogenen Dispersionen von 10 g Glycerin und 2 g Talk in ad 100 g Wasser und dann mit einer Zubereitung von 25 g Chitosan (Hydagen® DCMF, 1 Gew.-%ig in Glycolsäure, Cognis Deutschland GmbH & Co. KG), 4 g Salicylsäure, 4 g Glyzyrrhetinsäuresalicylester, Phenonip® (Konservierungsmittelmischung enthaltend Phenoxyethanol und Parabene) und 0,5 g Polysorbat-20 in ad 100 g Wasser versetzt. Die erhaltene Matrix wurde filtriert, auf 60 °C erwärmt und in eine 0,5 Gew.-%ige Natriumalginatlösung getropft. Nach dem Sieben wurde eine wässrige Zubereitung erhalten, die 8 Gew.-% Mikrokapseln mit einem mittleren Durchmesser von 1 mm enthielt.

**Beispiel 2.** In einem 500-ml-Dreihalskolben mit Rührer und Rückflusskühler wurden in der Siedehitze 3 g Agar-Agar in 200 ml Wasser gelöst. Anschließend wurde die Mischung innerhalb von etwa 30 min unter starkem Rühren zunächst mit einer homogenen Dispersionen von 10 g Glycerin und 2 g Talk in ad 100 g Wasser und dann mit einer Zubereitung von 25 g Chitosan, 4 g Glyzyrrhetinsäureretinylester, 0,5 g Phenonip® und 0,5 g Polysorbat-20 in ad 100 g Wasser versetzt. Die erhaltene Matrix wurde filtriert, auf 50 °C temperiert und unter starken Rühren im 2,5fachen Volumen Paraffinöl, das zuvor auf 15 °C gekühlt worden war, dispergiert. Die Dispersion wurde anschließend mit einer wässrigen Lösung enthaltend 1 Gew.-% Natriumlaurylsulfat und 0,5 Gew.-% Natriumalginat und dann mehrfach mit einer 0,5 Gew.-%igen wässrigen Phenoniplösung gewaschen, wobei die Ölphase entfernt wurde. Nach dem Sieben wurde eine wässrige Zubereitung erhalten, die 8 Gew.-% Mikrokapseln mit einem mittleren Durchmesser von 1 mm enthielt.

**Beispiel 3.** In einem 500-ml-Dreihalskolben mit Rührer und Rückflusskühler wurden in der Siedehitze 3 g Agar-Agar in 200 ml Wasser gelöst. Anschließend wurde die Mischung innerhalb von etwa 30 min unter starkem Rühren zunächst mit einer homogenen Dispersionen von 10 g Glycerin und 2 g Talk in ad 100 g Wasser und dann mit einer Zubereitung von 25 g Chitosan, 4 g Glyzyrrhetinsäuretocopherylester, 0,5 g Phenonip® und 0,5 g Polysorbat-20 in ad 100 g Wasser versetzt. Die erhaltene Matrix wurde filtriert, auf 60 °C erwärmt und in eine 15 Gew.-%ige Lösung von Sodium Laureth Sulfate getropft. Nach dem Sieben wurde eine wässrige Zubereitung erhalten, die 9 Gew.-% Mikrokapseln mit einem mittleren Durchmesser von 1 mm enthielt.

### Antibakterielle Wirksamkeit

Im folgenden wurden folgende Wirkstoffe bzw. deren Gemische getestet:
A1 Glyzyrrhetinsäure
A2 Salicylsäure
B1 Glyzyrrhetinsäuresalicylester
B2 Glyzyrrhetinsäureretinylester
B3 Glyzyrrhetinsäuretocopherylester

Die antibakterielle Wirksamkeit der Wirkstoffe und Wirkstoffmischungen wurde mit Hilfe der Diffusionsmethode auf Agar-Platten bzw. der Agar-Verdünnungsmethode untersucht. Dabei wird zunächst ein rundes Filterpapier definierten Durchmessers mit der Testlösung getränkt und dieses dann auf die Oberfläche einer Agarplatte aufgebracht, welche zuvor mit den Testmikroorganismen inokuliert worden war. Anschließend wird nach definierten Zeiten die Größe der Inhibierungszonen bestimmt. Diese Technik erlaubt es insbesondere die MIC (Minimum Inhibitory Concentration) als die niedrigste Testsubstanzkonzentration zu bestimmen, mit der noch eine vollständige Inhibierung der Mikroorganismen erzielt werden kann.

*Agar-Diffusionsmethode.* Das Inokulum wurde unter Verwendung einer frischen Kultur in einer stationären Wachstumsphase (nach ca. 18-24 h) in einer BHI-Lösung (Brain-Heart-InfusionI) hergestellt. Die Bakteriensuspension wurde auf 0,5 MacFarland-Einheiten eingestellt, entsprechend 1,5 10⁸ Kolonienbildende Einheiten (cfu)/ml; anschließend wurde die Suspension mit einer Kochsalzlösung 1/100 verdünnt, um einen Wert von 1,5 10⁶ cfu/ml einzustellen. Anschließend wurde Mueller-Hinton Agar *(Staphylococcus epidermis, Staphylococcus aureus)* und Wilkins-Chalgrens Agar mit 5 Gew.-% Schafsblut *(Propionobakterium acnes)* 15 min bei 121 °C sterilisiert und dann in Petrischalen gegeben. Die Petrischalen wurden mit 2 bis 4 ml der Bakteriensuspensionen versetzt und bei Raumtemperatur getrocknet. Die Filterpapiere wurden mit 20 µl der Testsubstanzen getränkt und auf die Oberfläche der Agarplatten aufgebracht. Die inokulierten Petrischalen wurden 18 bis 24 bei 37 °C inkubiert (die Petrischalen mit Propionibacterium acnes unter anaeroben Bedingungen) und die antimikrobielle Wirksamkeit anschließend durch Bestimmung der Wachstumsinhibierungszone ermittelt. Die Ergebnisse sind in der Wachstumsinhibierungszone ermittelt. Die Ergebnisse sind in Tabelle 1 zusammengefasst. Angegeben sind ist jeweils der Durchmesser der Inhibierungsflächen in mm.

*Agar Verdünnungsmethode (MIC-Bestimmung).* Wie oben beschrieben wurden verschiedene Agars vorbereitet, mit unterschiedlichen Konzentrationen an Testsubstanzen versetzt, homogenisiert und dann getrocknet. Anschließend erfolgte die Inokulation der Petrischalen mit jeweils 2 µl der Bakteriensuspensionen. Nach dem erneuten Trocknen wurden die Petrischalen bei 37 °C 18 bis 24 h inkubiert. In Tabelle 2 sind die MIC in mg/ml angegeben, d.h. die geringsten Konzentrationen, mit denen noch eine vollständige Inhibierung des Bakterienwachstums erreicht werden kann. Es handelt sich jeweils um den Mittelwert von Doppelbestimmungen.

**Tabelle 1**

| **Inhibierungszonen [mm]** | | | |
|---|---|---|---|
| **Wirkstoffverhältnis** | **Staphylococcus aureus** | **Staphylococcus epidermidis** | **Propionibacterium acnes** |
| A1 | 5 | 4 | 6 |
| A2 | 5 | 5 | 4 |
| B1 | 10 | 11 | 11 |
| B2 | 14 | 15 | 10 |
| B3 | 10 | 13 | 11 |
| B2 : A1 = 50 : 50 | 16 | 17 | 14 |
| B2 : A2 = 50 : 50 | 15 | 14 | 14 |

**Tabelle 2:**

| **MIC [mg/ml]** | | | |
|---|---|---|---|
| **Wirkstoffe** | **Staphylococcus epidermidis** | **Staphylococcus aureus** | **Propionibacterium acnes** |
| A1 | 1,25 | >10 | 1,25 |
| A2 | 1,25 | >10 | 1,25 |
| B1 | 1,25 | 1,25 | 0,625 |
| B2 | 0,625 | 1,25 | 0,625 |
| B3 | 1,25 | 1,25 | 0,625 |
| B2 : A1 = 50 : 50 | 1,25 | 1,25 | 0,625 |
| B2 : A2 = 50 : 50 | 0,625 | 1,25 | 0,625 |

Die Ergebnisse zeigen, dass die Glyzyrrhetinsäureester gegenüber den freien Säuren über verbesserte antimikrobielle Eigenschaften speziell gegenüber solchen Keimen verfügen, die in die Entstehung von Akne involviert sind. Diese Eigenschaften können in Abmischung noch verbessert werden.

## Patentansprüche

1. Ester der Glyzyrrhetinsäure gemäß Formel (I), 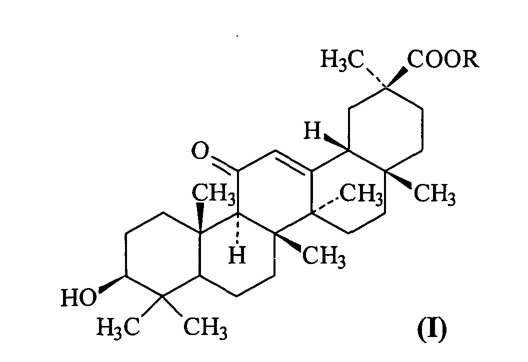 in der R für den Rest eines aromatischen Alkohols steht.

2. Ester nach Anspruch 1, **dadurch gekennzeichnet, dass** der aromatische Alkohol ausgewählt ist aus der Gruppe, die gebildet wird von Benzylalkohol, Salicylalkohol, Sterol, Tocopherol, Retinol sowie deren Gemischen.

3. Anti-Aknemittel, enthaltend eine wirksame Menge von Estern der Glyzyrrhetinsäure nach Anspruch 1.

4. Mittel nach Anspruch 3, **dadurch gekennzeichnet, dass** sie weiterhin Wirkstoffe enthalten, die ausgewählt sind aus der Gruppe, die gebildet wird von
• Glyzyrrhetinsäure und deren Salzen oder Extrakte der Pflanze *Glyzyrrhiza glabra,*
• Azelainsäure, deren Salzen und Estern,
• Salicylsäure, deren Salzen und Estern,
• Benzoylperoxid,
• Vitamin B6
• Vitamin E phosphat sowie
• Pflanzenextrakten.

5. Mittel nach den Ansprüchen 3 und/oder 4, **dadurch gekennzeichnet, dass** sie die Wirkstoffe in Form von Mikrokapseln oder Pro-Liposomen enthalten.

6. Verwendung von Estern der Glyzyrrhetinsäure nach den Ansprüchen 1 und/oder 2 zur Herstellung von kosmetischen, pharmazeutischen oder dermatologischen Zubereitungen.

7. Verwendung nach Anspruch 6, **dadurch gekennzeichnet, dass** die Zubereitungen Anti-Aknemittel darstellen.

8. Verwendung nach den Ansprüchen 6 und/oder 7, **dadurch gekennzeichnet, dass** die Zubereitungen die Ester in Mengen von 0,01 bis 20 Gew.-% enthalten.
